# EUROPEAN PATENT SPECIFICATION

(11) **EP 1 991 868 B1**
(45) Date of publication and mention of the grant of the patent: **15.09.2010**
(21) Application number: 07704362.8
(22) Date of filing: 05.02.2007
(51) Int. Cl.: G01N 33/50

(54) **METHOD TO DETERMINE PSEUDO-ALLERGIC REACTIONS**
VERFAHREN ZUR BESTIMMUNG PSEUDOALLERGISCHER REAKTIONEN
MÉTHODE POUR DÉTERMINER DES RÉACTIONS PSEUDO-ALLERGIQUES

(30) Priority: 16.02.2006 EP 06110056
(43) Date of publication of application: 19.11.2008
(73) Proprietor: Bracco Suisse SA, 6928 Manno (CH)
(72) Inventor: FOUILLET, Xavier, CH-1228 Plan-les-Ouates (CH)
(74) Representative: Ravizza, Claudio
(86) International application number: PCT/EP2007/051078
(87) International publication number: WO 2007/093517

(56) References cited:
- FR-A- 2 765 341
- US-A1- 2004 265 925
- BOUMIZA R ET AL: "Marked improvement of the basophil activation test by detecting CD203c instead of CD63" CLINICAL AND EXPERIMENTAL ALLERGY, BLACKWELL SCIENTIFIC PUBLICATIONS, LONDON, GB, vol. 33, no. 2, February 2003 (2003-02), pages 259-265, XP002245136 ISSN: 0954-7894 cited in the application
- BOUMIZA RADHIA ET AL: "The basophil activation test by flow cytometry: recent developments in clinical studies, standardization and emerging perspectives." CLINICAL AND MOLECULAR ALLERGY [ELECTRONIC RESOURCE] : CMA. 30 JUN 2005, ARTICLE N.9, vol. 3, 30 June 2005 (2005-06-30), XP002376247 ISSN: 1476-7961 cited in the application
- SZEBENI ET AL.: "Animal models of complement-mediated hypersensitivity reactions to liposomes and other lipid-based nanoparticles." JOURNAL OF LIPOSOME RESEARCH, vol. 17, 2007, pages 107-117,
- DE WECK A. L.: "PATHOPHYSIOLOGIC MECHANISMS OF ALLERGIC AND PSEUDO-ALLERGIC REACTIONS TO FOODS FOOD ADDITIVES AND DRUGS" ANNALS OF ALLERGY, vol. 53, 1984, pages 583-586, XP008114326

## Description

### Technical field

The invention relates to a method and to a kit for determining the possible appearance of adverse reactions (such as anaphylactoid reactions) in patients in need to undergo to an administration of a pharmaceutical compound.

### Background of the invention

The incidence of adverse reactions in patients receiving a pharmaceutical compound, particularly by injection thereof, is rather significant. Of particular importance are pseudo-allergic reactions (also known as non-allergic hypersensitivity) related to complement activation, which are often classified as "Complement Activation-Related Pseudo-Allergy" reactions (in short "CARPA" reactions). These reactions have been described with particular reference to the administration of contrast agents and intravenously administered drugs by J. Szebeni et al. in the articles: "Hypersensitivity reactions to radiocontrast media: the role of complement activation", Curr.Allergy Asthma Rep. 4 (2004), 25-30, "Complement activation-related pseudoallergy caused by liposomes, micellar carriers of intravenous drugs, and radiocontrast agents", Crit Rev.Ther.Drug Carrier Syst. 18 (2001), 567-606 and " Complement activation-related pseudoallergy: a new class of drug-induced acute immune toxicity" Toxicology. 216 (2005) 106-21.

Typical methods to determine possible adverse reactions caused by administration of a pharmaceutical compound involve skin test, where an antigen is administered to the skin of the patient (e.g. by puncture or subcutaneous injection) in low doses and possible skin reactions are observed.

An in vitro test has recently been developed (see articles of R. Boumiza et al.: "Marked improvement of the basophil activation test by detecting CD203c instead of CD63", Clin.Exp.Allergy 33 (2003), 259-265, and "The basophil activation test by flow cytometry: recent improvements and emerging perspectives" Clin. Mol. Allergy 2005, 3:9) to predict the potential allergenic effect of a drug. According to this test, also known as the "basophil activation test", a small amount of blood is incubated in vitro with the substance of which the potential allergenic effect has to be evaluated; CD203c, a surface antigen selectively expressed on human basophils, is used as a marker to predict the potential allergenic effect of the drug. As mentioned in said articles, CD203c corresponds to a surface antigen expressed on human basophils recently recognized by the monoclonal antibody 97A6. This antigen, belonging to the type II transmembrane protein family, is a multifunctional ectoenzyme called ectonucleotide pyrophosphatase phophodiesterase 3 (E-NPP3) that catalyzes the cleavage of a number of molecules including deoxynucleotides and nucleotide sugars. The expression of this marker on the basophils is rapidly upregulated upon exposure to an allergen. A commercial kit to carry out this method is available from Immunotech (Beckman Coulter), France, under the name Allerginicity kit - Cellular Analysis of Allergy. This approach has however the disadvantage that the test has to be performed for each pharmaceutical compound to be administered, for assessing Its possible allergenic effects. In addition, it does not provide any clear evidence of possible pseudo-allergic reactions in a patient subjected to the drug administration.

According to another approach disclosed in US patent application 2005/0220708, in vivo detection of non-allergic hypersensitivity of a subject is effected by Intradermal or cutaneous administration of an anaphylatoxin to the subject. According to this approach, a (skin) reaction to said administration is indicative of a non-allergic hypersensitivity of the subject. As for other in vivo allergenic tests, this test may however be contraindicated for subjects at risk of important hypersensitivity reactions; in addition, skin reactions may often be difficult to evaluate and/or quantify.

US patent application 2004/0265 relates to a method for measuring the activation of basophils upon stimulation with an allergen, to determine possible hypersensitivity of a subject to some substances.

French patent application 2 765 341 discloses a method for determining human basophils activation, by measuring the expression of CD63 marker on basophils membrane.

Boumiza R. et al "Marked improvement of basophil activation test by detecting CD203c instead of CD63", Clin. Exp. Allergy, 2003, 33:259-265, discloses a method for determining basophils activation based on the expression of CD203c marker on basophils membrane.

### Summary of the invention

The Applicant has now found a new method for the in vitro determination of possible pseudo-allergic reactions in a subject.

The present invention is based on the finding that anaphylatoxins (which are typically released during complement activation in the body) may activate the basophil blood cells and that said activation can be measured. In particular, said anaphylatoxins may upregulate the expression of some specific markers on the surface of basophil cells, which markers can then be quantified according to conventional analytical techniques. Results showing a more or less high activation of basophil cells may then be useful to predict more or less important pseudo-allergenic reactions in a patient related to complement activation.

A first aspect of the invention relates to a method for determining potential hypersensitivity to pseudo-allergic reactions in a subject, which comprises:
- adding a predetermined amount of C3A, C5A or mixtures thereof to a sample of complete blood of the subject; and
- determining the amount of activation of the basophil cells in said blood sample.
Preferably, the determination of said activation is carried out by measuring the expression of an activation marker on the surface of said basophil cells. Preferably, said activation marker is CD203c or CD63, more preferably CD203c.

Said determination is preferably made by means of an antibody capable of specifically binding to said activation marker. Preferably said antibody is an antibody bearing a fluorescent tracer. Preferably said determination is implemented by flow cytometry analysis of the basophil cells.

According to a preferred embodiment, the anaphylatoxin is selected from C3a, C5a and mixtures thereof.

### Detailed description of the invention

According to the present invention, it is possible to evaluate possible pseudo allergic reactions induced by complement activation in a subject, before the same undergoes to the administration of a pharmaceutical compound. The test is easily accomplished in vitro, without any discomfort for the subject.

The term "subject" refers to a living subject (including human beings) for whom a likelihood of pseudo-allergic reactions has to be assessed, typically before administering to the same any pharmaceutical compound which may provoke said pseudo-allergic reaction.

The method entails the use of the anaphilatoxins selected from C3A, C5A and mixtures thereof, which is added to a sample of complete blood of a subject, to determine said possible pseudo-allergic reaction in the subject.

The expression "complete blood" indicates that the sample of blood under examination does not undergo to any particular separation or enrichment procedure of its components, before its admixing with the compound having anaphylatoxic activity. Said blood sample will thus have substantially the same composition of the blood in the subject, in particular with respect to the concentration of red and white blood cells and to the composition of the plasma.

Typically, anaphylatoxins are peptide fragments that are produced during the pathways of the complement system. The complement system is derived from many small plasma proteins that form the complex biochemical cascade of the immune system; activation of this system leads to cytolysis, chemotaxis, opsonization and inflammation, as well as the marking of pathogens for phagocytosis. A notable feature of anaphylatoxins is their ability to trigger degranulation of (release of substances from) mast cells and basophils, typically with release of histamine. Examples of analogues or derivatives of an anaphylatoxin are, for instance, natural or synthetic peptides based on the structure of human C3a or C5a. Other examples are C3a or C5a analogue molecules mimicking the activity of human C3a or C5a anaphylatoxins. Examples of peptides with anaphylatoxic activity are disclosed, for instance, in the above cited US patent application 2005/0220708, the content of which is herein incorporated by reference. Other anaphylatoxins analogues may include organic small molecules capable of binding and stimulating C3a and C5a receptors on basophils. In some applications, it may also be useful to use non-human complement fragments with anaphylatoxic activity, as well as analogues and derivatives thereof, such as, for instance, rat C3a or C5a or bovine C5a. Preferably human C3a, C5a or mixtures thereof are employed, more preferably C5a. C5a anaphylatoxin is commercially available from suppliers of biochemical products, such as Cell Sciences Inc., Sigma-Aldrich Corp. or Merck Biosciences (Calbiochem).

According to a preferred embodiment the first step of a method according to the invention comprises incubating C3A, C5A or mixtures thereof, with a sample of complete blood taken from a subject. The blood sampling is preferably performed by finger puncture, although it could also be done by venipuncture. Since EDTA may inhibit complement activation, in order to not interfere with the phenomenon of basophil activation by the complement anaphylatoxin, blood is preferably collected on a non-EDTA anticoagulant. Examples of non-EDTA anti-coagulants are hirudin or citrate. Preferably, hirudin is employed as anticoagulant. The final concentration of hirudin anticoagulant is from about 800 ATU/ml of blood to about 1200 ATU/ml of blood, preferably of about 1000 ATU of anticoagulant per ml of blood (ATU=Anti-thrombotic unit).

C3A, C5A or mixtures thereof is then added to a blood sample containing the anticoagulant and after further addition of a reactive for quantifying the amount of activated basophils, the mixture is incubated at 37 °C in the dark. Optionally, a saline solution (e.g. PBS, i.e. phosphate buffer solution) can be added to the mixture in order to adjust the content of each tube of the series to the same volume.

The concentration of C3A, C5A or the mixtures thereof in the assay tube is preferably of from about 5.0-10⁻¹⁰ M to about 5.0.10⁻⁸ M, more preferably of from about 1.010⁻⁹ M to about 1.0·10⁻⁸ M. According to alternative embodiments, a plurality of tubes (e.g. about 2 to 5) containing different amounts of the selected anaphylatoxin can be prepared in order to evaluate the effects of different concentrations thereof. For instance, three different tubes can be prepared with increasing concentrations of anaphylatoxins of 6.0·10⁻¹⁰ M, 1.5·10⁻⁹ M and 2.2·10⁻⁹ M respectively. The effective amounts of anaphylatoxin will depend from various parameters, including, but not limited to, the basophil activation activity of the anaphylatoxin employed and/or the patient subjected to the evaluation.

Preferably, comparative positive and negative controls are also prepared, in order to better quantify the pseudo-allergic reaction. Negative controls can be prepared by adding only a saline solution (e.g. PBS) to a tube. The negative control typically shows an amount of spontaneously activated basophils in the order of about 1-3% of the total number. Positive controls can be prepared by adding (instead of the anaphylatoxin) an antigen known to activate the basophils. For instance an anti-immunoglobuline E (anti-IgE) can be added to the mixture in the positive control tube. Positive controls should activate about 80-90% of the total number of basophils.

The reactive which is added to the mixture in order to detect and quantify the amount of activated basophils can be any compound known in the art for selectively binding to markers on the basophil cell surface (which are expressed when the basophils are activated by the complement peptides) and for being then detectable by means of conventional analytical techniques. For instance, the reactive can be a fluorescent antibody to the CD203c marker. CD203c corresponds to a surface antigen specifically expressed on cells from the basophil/mastocytes lineage in humans.

The antibody is preferably conjugated with a suitable tracing molecule, i.e. a molecule capable of being detected with a respective analytical system. For instance, the tracing molecule in the case of flow cytometry can advantageously be a fluorescent marker, such as fluoresceine isothyocyanate (FITC), R phycoerythrin (PE) or R phycoerytrin convalently bound to cyanine (PC7). Preferably, said reactive is a three-colour reactive for flow cytometric protocol for monitoring basophil activation, in particular for staining CRTH2, CD3 and CD203c markers on eosinophils, basophils and/or Th2 lymphocytes. In particular, CRTH2 staining allows CRTH2-expressing cells (eosinophils, basophils and Th2 lymphocytes) to easily be distinguished from other cells. On the basis of light scattering, eosinophils are easily excluded from the analysis. Basophils could then readily be distinguished from Th2 lymphocytes on the basis of CD3, staining, as this marker is not present on basophils. Finally, on this gated population of basophils (low light scatterings, CRTH2-positive and CD3-negative), modulation of CD203c can be monitored upon challenge with the anaphylatoxin. Advantageously, said three-colour reactive comprises the three fluorescent antibodies PE-CD203c, FITC-CRTH2 and PC7-CD3 (with respective monoclonal antibodies BM16, 97A6 and UCHT1), as described in the protocol of the Allergenicity test commercialized by Immunochem (Beckman Coulter). As mentioned in the guidelines of said test, the 3-color combination is a mixture of 2-fluorescent murine monoclonal antibodies (CD203c-PE and CD3-PC7) and one-fluorescent rat monoclonal antibody (CRTH2-FITC). The monoclonal antibody (mAb) BM16 precipitates a 55 to 70 kDa protein from cells lysates of CRTH2-transfected JURKAT and from established Th2 clone, (e.g. clone 6L21) corresponding to PGD2 receptor; MAb 97A6 has been assigned to the CD203c cluster of differentiation; while MAb UCHT1, reacting with the ε chain of the CD3 complex, has been assigned to CD3. Depending on the specific marker to be detected and on the specific analytical technique applied, those skilled in the art, once armed with the present description, may easily select the most suitable antibody or group of antibodies and the corresponding tracing molecule(s).

The mixtures are then incubated under conventional conditions (e.g. for about 10 to 30 minutes, typically about 15 minutes, at a temperature of about 36-38°C).

After incubation, red blood cells (erythrocytes) are hemolyzed (e.g. by means of a hemolyzing solution or distilled water) and the white blood cells (leukocytes) are fixed (e.g. by adding paraformaldehyde). The content of the tubes is then centrifuged and rinsed with PBS and the obtained suspension is analyzed by flow cytometry. The detection and quantification of non-specific activation of the patient's blood basophils by the anaphylatoxin can be carried out by any conventional technique known in the art, depending on the specific reactive employed for the detection and quantification of activated basophils. For instance, when a fluorescent antibody is employed, the analysis can be implemented by means of flow cytometry, e.g. by using a FACS (fluorescent-activated cell sorter) machine. For instance, the detection and quantification protocol described in the above cited Allergenicity Test from Immunochem can be used.

The assessment of the results of the test can be carried out according to conventional protocols in the art. Thus, for instance, for "healthy" patients (i.e. subjects which will unlikely show an important pseudo allergic reactions upon administration of a pharmaceutical compound), the amount of activated basophils in the presence of anaphylatoxin will correspond to the amount of activated basophils measured in the negative control test, or will be slightly higher (e.g. less than 2 to 3-fold). On the contrary, the detection of higher amounts of activated basophils, also depending from e.g. the ethnic group, age, gender and medical history of the patient, may be indicative of more or less severe potential for pseudo allergic reactions. For instance, the activation of 5 or 10 times the amount of basophils activated in the negative control may be indicative of a potential for pseudo allergic reaction in the patient.

The method above described can be useful for assessing potential pseudo-allergic reactions (or "CARPA" reactions) in a patient in need to undergo to an administration of a pharmaceutical component, particularly by injection (e.g. parenterally or intravenously) thereof and, more in general, of any substance which, when administered to the patient, may cause a CARPA reaction. The term pharmaceutical component comprises within its meaning any substance or composition which may have a biological, therapeutic and/or diagnostic effect on a subject to which it is administered, thus including, for instance: biological material, such as proteins (including e.g. recombinant plasma proteins, coagulation/haemostatic proteins, immunoglobulins or complement proteins), blood substitutes or proteinase inhibitor molecules; drugs, particularly administered by infusion, including drugs for tumoral treatment; contrast agents, such as X-ray contrast agents, MRI contrast agents and ultrasound contrast agents (including, for instance, gas-filled microvesicles stabilized by a phospholipid layer).

The following prophetic examples will help to further illustrate the invention.

### EXAMPLES

### Materials:

Anaphylatoxin: complement C5a human, from Sigma-Aldrich;
Three-colour antibody fluorescent reactive for detection of basophils activation and anti-IgE positive control, from Immunochem (components of Allerginicity kit).

Within less than 2 hours before running the test, quantities of about 200 µL of blood (collected from a patient by finger puncture) are poured into Eppendorf tubes containing ten (10) µL of a solution of hirudin in saline at 20 ATU/µL. The final concentration of hirudin is of about 1000 ATU/mL blood. A total of 3 Eppendorf tubes of 200 µL of hirudinated blood per patient is needed to run the test. If more practical, 2 mL blood can be collected by venipucture on 100 µL hirudin at 20 ATU/mL in order to obtain a final concentration of 1000 ATU/ml blood

Then five identical test tubes, marked 1 to 5, are prepared to run the process. To each of the above tubes, the following components are respectively added:
Tube 1 (negative control): 20 µL of phosphate buffered saline (PBS);
Tube 2 (positive control): 20 µL of anti-IgE reagent which will induce a high level of basophil activation;
Tube 3: 50 µL of PBS solution containing C5a anaphylatoxin at a concentration of 200 ng/ml;

Twenty (20) µL of fluorescent reactive, 100 µL of anticoagulated blood and complementary volumes of PBS solution (up to a total volume of 220 µL in each tube) are then added to each tube. Tubes are then incubated for 15 min at 37 °C in the dark. After incubation, a hemolyzing solution is added to the test tube to hemolyze erythrocytes, then a solution of paraformaldehyde in PBS is added to fix the leukocytes. The test tubes are centrifugated and rinsed twice with PBS to eliminate the red blood cell debris and hemoglobin which would interfere with the analysis. A suspension of fluorescent leukocytes in a total volume of about 500 µL for each tube is finally recovered (containing about 2% of basophils), which is then are analyzed by flow cytometry.

The leukocyte suspension is then analyzed by means of a FACS machine (beckon Dickinson FACSort, in Facscalibur^{™} configuration). The results are gated from the same gate using the FACS machine software for all the tubes of the same experiment.

By analyzing the histogram of the suspensions in tube 1 (negative control) an amount of activated basophil cells lower than 3% is observed, while the histogram of tube 2 (positive control) shows an amount of activated basophils higher than 95%.

An amount of activated basophil cells higher than about 15% measured in the test tube 3 can be indicative of a possible risk of CARPA reactions in the patient. Higher values of basophiles activation will respectively be indicative of the possibility of more severe CARPA reactions in the patient.

Alternatively, a plurality of tubes containing different amounts of anaphylatoxin can be tested. For instance, in the above test, three tubes (tubes 3, 4 and 5) can be prepared, containing a respective amount of about 4 ng, 10 ng and 15 ng of C5a anaphylatoxin (in the final volume of 220 µl).

The lowest amount of anaphylatoxin (tube 3) can be chosen in order to obtain results similar or slightly superior to the negative control values from healthy donors (i.e. showing substantially no hypersensitivity) in a determined group or population of patients. This value is thus taken as a basis for assessing a negative response in said group or population. The curve determined by the response of basophil activation with respect to the various concentrations of anaphylatoxin can then be used to assess the possible risk of a CARPA reaction in a patient (e.g. steeper curves will correspond to a higher risk).

## Claims

1. Method for determining potential hypersensitivity to pseudo-allergic reactions in a subject, which comprises:
- adding a predetermined amount of an anaphylatoxin selected from C3a, C5a, or mixtures thereof, to a sample of complete blood of said subject; and
- determining the amount of activation of the basophil cells in said blood sample.

2. Method according to claim 1 wherein said step of determining the amount of basophile activation is carried out by measuring the expression of an activation marker on the surface of said basophil cells.

3. Method according to claim 2 wherein said cell marker is CD203c or CD63.

4. Method according to claims 1 or 2 wherein said determination of basophils activation is made by means of an antibody capable of specifically binding to said activation marker.

5. Method according to claim 4 wherein said antibody comprises a fluorescent tracer.

6. Method according to any of the preceding claims wherein said determination of basophile activation is carried out by flow cytometry analysis.

7. Method according to any of the preceding claims wherein the amount of anaphylatoxin is of from 5.0·10⁻¹⁰ M to 5.0·10⁻⁸ M.

8. Method according to claim 7, wherein said amount is of from 1.0·10⁻⁹ M to 1.0·10⁻⁸M.

9. Method according to claim 1, wherein the amount of activated basophils measured in the sample of complete blood of said subject is compared with the amount of activated basophils determined in a negative control.

10. Method according to claim 9 wherein said negative control is prepared by admixing a sample of complete blood of said subject with a saline solution.

11. Method according to claims 9 or 10 wherein the amount of activated basophils in said negative control is 1-3% of the total basophil numbers.

12. Method according to claims 9, 10 or 11, wherein a five times higher amount of actived basophils in the sample of complete blood of said subject, compared to the amount of activated basophils in the negative control, may be indicative of potential hypersensitivity to pseudo-allergic reactions in said subject.

13. Method according to claims 9, 10 or 11, which further comprises comparing the amount of activated basophils measured in the sample of complete blood of said subject with the amount of activated basophils determined in a positive control.

14. Method according to claim 13, wherein said positive control is prepared by admixing a sample of complete blood of said subject with an immunoglobulin E.

15. Method according to claims 13 or 14 wherein said amount of activated basophils in the positive control is of about 80-90% of total basophil numbers.

## Patentansprüche

1. Verfahren zur Bestimmung einer potenziellen Überempfindlichkeit gegen pseudo-allergische Reaktionen in einem Probanden, bei dem man
- eine vorbestimmte Menge eines Anaphylatoxins ausgewählt unter C3a, C5a oder Mischungen davon zu einer Vollblutprobe des Probanden hinzugibt und
- man den Umfang der Aktivierung der basophilen Zellen in der Blutprobe bestimmt.

2. Verfahren nach Anspruch 1, bei dem man den Umfang der basophilen Aktivierung durch Messen der Expression eines Aktivierungsmarkers auf der Oberfläche der basophilen Zellen bestimmt .

3. Verfahren nach Anspruch 2, bei dem der Zellmarker CD203c oder CD63 ist.

4. Verfahren nach den Ansprüchen 1 oder 2, bei dem man die Bestimmung der basophilen Aktivierung mit Hilfe von Antikörpern durchführt, die spezifisch an die Aktivierungsmarker binden können.

5. Verfahren nach Anspruch 4, wobei die Antikörper einen fluoreszierenden Tracer enthalten.

6. Verfahren nach einem der vorangehenden Ansprüche, bei dem man die Bestimmung der basophilen Aktivierung durch eine durchflusszytometrische Analyse durchführt.

7. Verfahren nach einem der vorangehenden Ansprüche, bei dem die Menge an Anaphylatoxin von 5,0 x 10⁻¹⁰ M bis 5,0 x 10⁻⁸ M beträgt.

8. Verfahren nach Anspruch 7, bei dem die Menge von 1,0 x 10⁻⁹ M bis 1,0 x 10⁻⁸ M beträgt.

9. Verfahren nach Anspruch 1, bei dem man die in der Vollblutprobe des Probanden gemessene Menge an aktivierten Basophilen mit der Menge an aktivierten Basophilen vergleicht, die in einer negativen Kontrolle bestimmt wurden.

10. Verfahren nach Anspruch 9, bei dem man die negative Kontrolle durch Mischen einer Vollblutprobe des Probanden mit einer Salzlösung herstellt.

11. Verfahren nach den Ansprüchen 9 oder 10, bei dem die Menge an aktivierten Basophilen in der negativen Kontrolle 1 bis 3 % der Gesamtbasophilzahl beträgt.

12. Verfahren nach den Ansprüchen 9, 10 oder 11, bei dem eine 5-fach höhere Menge an aktivierten Basophilen in der Vollblutprobe des Probanden im Vergleich zur Menge an aktivierten Basophilen in der negativen Kontrolle ein Hinweis auf eine potenzielle Überempfindlichkeit gegen pseudo-allergische Reaktionen des Probanden sein kann.

13. Verfahren nach den Ansprüchen 9, 10 oder 11, bei dem man weiterhin die in der Vollblutprobe des Probanden gemessene Menge an aktivierten Basophilen mit der Menge an aktivierten Basophilen vergleicht, die in einer positiven Kontrolle bestimmt wurde.

14. Verfahren nach Anspruch 13, bei dem man die positive Kontrolle durch Mischen einer Vollblutprobe des Probanden mit einem Immunoglobulin E herstellt.

15. Verfahren nach den Ansprüchen 13 oder 14, bei dem die Menge an aktivierten Basophilen in der positiven Kontrolle etwa 80 bis 90 % der Gesamtbasophilzahl beträgt.

## Revendications

1. Procédé permettant de déterminer, chez un sujet, une potentielle hypersensibilité à des réactions pseudo-allergiques, lequel procédé comporte les étapes suivantes :
- ajouter, à un échantillon de sang complet dudit sujet, une quantité prédéterminée d'une anaphylatoxine choisie parmi les toxines C3a et C5a et leurs mélanges,
- et déterminer le degré d'activation des basophiles dans ledit échantillon de sang.

2. Procédé conforme à la revendication 1, dans lequel on effectue ladite étape consistant à déterminer le degré d'activation des basophiles en évaluant l'expression d'un marqueur d'activation à la surface desdits basophiles.

3. Procédé conforme à la revendication 2, dans lequel ledit marqueur cellulaire est CD203c ou CD63.

4. Procédé conforme à la revendication 1 ou 2, dans lequel ladite détermination du degré d'activation des basophiles est réalisée au moyen d'un anticorps capable de se lier spécifiquement audit marqueur d'activation.

5. Procédé conforme à la revendication 4, dans lequel ledit anticorps comporte un traceur fluorescent.

6. Procédé conforme à l'une des revendications précédentes, dans lequel ladite détermination du degré d'activation des basophiles est réalisée par analyse en cytométrie de flux.

7. Procédé conforme à l'une des revendications précédentes, dans lequel la quantité d'anaphylatoxine vaut de 5,0.10⁻¹⁰ M à 5,0.10⁻⁸ M.

8. Procédé conforme à la revendication 7, dans lequel ladite quantité vaut de 1,0.10⁻⁹ M à 1,0.10⁻⁸ M_{.}

9. Procédé conforme à la revendication 1, dans lequel la quantité de basophiles activés mesurée dans l'échantillon de sang complet dudit sujet est comparée avec la quantité de basophiles activés déterminée dans un échantillon témoin négatif.

10. Procédé conforme à la revendication 9, pour lequel on prépare ledit échantillon témoin négatif en mélangeant un échantillon de sang complet dudit sujet avec une solution salée.

11. Procédé conforme à la revendication 9 ou 10, dans lequel la quantité de basophiles activés dans ledit échantillon témoin négatif représente 1 à 3 % du nombre total de basophiles.

12. Procédé conforme à la revendication 9, 10 ou 11, dans lequel une quantité de basophiles activés dans l'échantillon de sang complet dudit sujet cinq fois plus grande que la quantité de basophiles activés dans l'échantillon témoin négatif peut indiquer chez ledit sujet une potentielle hypersensibilité à des réactions pseudo-allergiques.

13. Procédé conforme à la revendication 9, 10 ou 11, qui comporte en outre le fait de comparer la quantité de basophiles activés mesurée dans l'échantillon de sang complet dudit sujet avec la quantité de basophiles activés déterminée dans un échantillon témoin positif.

14. Procédé conforme à la revendication 13, pour lequel on prépare ledit échantillon témoin positif en mélangeant un échantillon de sang complet dudit sujet avec une immunoglobuline E.

15. Procédé conforme à la revendication 13 ou 14, dans lequel la quantité de basophiles activés dans ledit échantillon témoin positif représente à peu près 80 à 90 % du nombre total de basophiles.
